# EUROPÄISCHE PATENTSCHRIFT

(11) **EP 2 108 331 B1**
(45) Veröffentlichungstag und Bekanntmachung des Hinweises auf die Patenterteilung: **21.12.2016**
(21) Anmeldenummer: 09008101.9
(22) Anmeldetag: 15.08.2003
(51) Int. Cl.: A61C 8/00, A61B 17/68, A61L 27/50

(54) **Implantat zur Implantation in Knochengewebe oder in durch Knochenersatzmaterial ergänztem Knochengewebe**
Implant for implantation in bone tissue or bone tissue supplemented with bone replacement material
Implant destiné à l'implantation dans du tissu osseux ou dans du tissu osseux remplacé par du matériau de remplacement d'os

(30) Priorität: 23.08.2002 CH 14522002
(43) Veröffentlichungstag der Anmeldung: 14.10.2009
(62) Teilanmeldung aus: 03792085.7
(73) Patentinhaber: Woodwelding AG, 6304 Zug (CH)
(72) Erfinder: Mayer, Jörg, 5702 Niederlenz (CH); Aeschlimann, Marcel, 2514 Ligerz (CH); Torriani, Laurent, 2516 Lamboing (CH)
(74) Vertreter: Frei Patent Attorneys

(56) Entgegenhaltungen:
- EP-A- 0 358 601
- WO-A-02/069817
- DE-A- 19 741 087
- SU-A1- 929 072
- US-A- 5 163 960
- US-A- 6 080 161

## Beschreibung

Die Erfindung liegt auf dem Gebiete der Medizinaltechnik und betrifft ein Implantat gemäss dem Anspruch 1. Das Implantat wird insbesondere in menschlichem oder tierischem Knochengewebe implantiert, kann aber auch in durch Knochenersatzmaterial ergänztem Knochengewebe implantiert werden.

Das erfindungsgemässe Implantat ist beispielsweise ein Dentalimplantat, das die Funktion einer natürlichen Zahnwurzel übernehmend in einen Kieferknochen eingesetzt wird und beispielsweise zur Befestigung einer künstlichen Zahnkrone, einer Brücke oder einer Zahnprothese an seinem proximalen Ende eine Fixierstelle aufweist, die nach der Implantation im Bereiche der Knochenoberfläche angeordnet ist. Das Dentalimplantat kann auch einen vollständigen Zahnersatz darstellen, das heisst zusätzlich zu einem zu implantierenden Wurzelbereich auch einen Kronenbereich aufweisen. Das Implantat kann aber auch eine andere Funktion haben und für die Implantation in einen anderen menschlichen oder tierischen Knochen geeignet sein. Das Implantat kann also ganz allgemein zur Verbindung eines Knochenteils mit einem anderen Gewebeteil, insbesondere Knochenteil, dienen oder mit einem künstlichen Teil, wobei der künstliche Teil einen Knochenteil stützen oder ersetzen kann (z.B. künstliche Gelenkkugel oder Gelenkpfanne) oder eine therapeutische Hilfsvorrichtung (z.B. Drug-release-Vorrichtung, Drainagevorrichtung oder Stimulator zur elektrischen oder chemischen Stimulation) ist. Das Implantat kann aber auch selbst eine solche therapeutische Hilfsvorrichtung sein. Es kann auch als Platzhalter für fehlendes und gegebenenfalls zu ersetzendes Knochengewebe dienen (z.B. nach Entfernung eines Tumors) oder als Augmentationselement für eine erwünschte Knochenvergrösserung.

Zahnersatzstrukturen (Einzelne Zähne, Zahngruppen, Teilprothesen oder ganze Prothesen), die auf den oben genannten Dentalimplantaten mit Fixierstellen basieren, werden gemäss dem Stande der Technik beispielsweise in den folgenden Schritten erstellt: Nach dem Entfernen der natürlichen Zahnwurzel wird gewartet, bis die Öffnung im Kieferknochen sich durch natürliche Knochenregeneration mit Knochengewebe gefüllt hat. Im Bereiche des regenerierten Knochengewebes wird eine an das Implantat angepasste Öffnung erstellt. Das Implantat wird in die Öffnung eingesetzt, wobei die Öffnung derart tief ist, dass das Implantat ganz darin Platz findet, die Öffnung also nicht überragt. Ein die Fixierstelle definierendes Innengewinde an der Stirnseite des Implantates wird mit einer Deckschraube abgeschlossen. Das Zahnfleisch wird über der Deckschraube zusammengenäht und es wird gewartet, bis das Knochengewebe mit dem Implantat verwachsen ist und dadurch eine für die zu erwartende Belastung genügende Stabilität (Sekundärstabilität) aufweist. Dann wird in einem weiteren Schritt das Zahnfleisch über dem Implantat geöffnet und die Deckschraube durch einen Distanzhalter ersetzt, wobei der Distanzhalter das Zahnfleisch überragt. Erst wenn das Zahnfleisch rund um den Distanzhalter verheilt ist, wird auf diesem die Zahnersatzstruktur befestigt. Das kurz beschriebene Prozedere bedeutet für den Patienten eine Behandlungszeit von bis zu 12 bis 18 Monaten. Davon fallen zwei bis drei Monate auf die Zeit zwischen der Implantation und einen Zeitpunkt, in dem das Implantat für eine Belastung genügend mit dem Knochengewebe verwachsen oder umwachsen ist.

Die erste Wartezeit (Regeneration des Knochengewebes in einer Öffnung im Kieferknochen) kann vermieden oder verkürzt werden, wenn Implantate verwendet werden, die in ihrer Form möglichst genau an die ursprüngliche Öffnung angepasst sind, wie dies beispielsweise in der Publikation US-6132214 (Suhonen et al.) beschrieben ist.

Die gemäss dem Stande der Technik verwendeten Dentalimplantate bestehen üblicherweise aus Reintitan oder Titanlegierungen. Diese Metalle zeigen eine sehr hohe biologische Verträglichkeit und es sind auch verschiedene Methoden bekannt, die Oberflächen von Implantaten aus diesen Metallen für eine noch verbesserte Osseointegration auszugestalten. Vielfach weisen die Implantate auch makroskopische Strukturen auf, die ein Einwachsen des Knochengewebes oder ein Durchwachsen mit Knochengewebe ermöglichen. Diese bekannten Dentalimplantate haben erst nach vollständiger Osseointegration eine Stabilität, die für eine volle Belastung reicht, also erst dann, wenn sie vom Knochengewebe eng umwachsen, bzw. mit dem Knochengewebe verwachsen oder von Knochengewebe durchwachsen sind (sekundäre Stabilität). In osteoporotischem oder weichem Knochen, sowie in schlecht regenerierendem Knochengewebe beispielsweise von älteren Patienten kann gegebenenfalls überhaupt keine genügende Implantatstabilität erreicht werden.

Die primäre Stabilität der oben beschriebenen Dentalimplantate, das heisst ihre Stabilität unmittelbar nach der Implantation ist sehr beschränkt. Aus diesem Grund wird die oben erwähnte Wartezeit zwischen Implantation und weiterem Aufbau eingeschoben. Die Primärstabilität der genannten Implantate ist je nach Implantatform verschieden aber häufig nicht für eine volle Belastung genügend. Stiftförmige Implantate mit einem Gewinde sind beschränkt auf Zug und Druck und mit Querkräften belastbar, insbesondere, wenn sie derart eingesetzt sind, dass mindestens ein Gewindegang im Bereich der Kortikalis liegt. Auf Torsion sind sie wenig belastbar. Implantate, die einen nicht runden Querschnitt haben, die also beispielsweise in ihrer Form an eine natürliche Zahnwurzel angepasst sind, sind auf Torsion besser belastbar, auf Zug weniger. Dasselbe gilt für plattenförmige Dentalimplantate, die auch mehrere Fixierstellen aufweisen können.

Die ungenügende Primärstabilität bekannter Dentalimplantate würde bei Belastung unmittelbar nach der Implantation zu derart starken Bewegungen zwischen Implantat und Knochengewebe führen, dass eine Osseointegration behindert oder sogar verhindert würde. Eine sofortige Belastung der Implantate wäre aber nicht nur wünschenswert, um die Behandlungszeit zu verkürzen sondern auch um einen durch Nichtbelastung bedingten Abbau des Kieferknochens zu vermeiden und um die Osseointegration zu fördern durch belastungsbedingte Mikrobewegungen zwischen Implantat und Knochengewebe, die aber ein physiologisches Mass nicht überschreiten dürfen.

Die primäre Stabilität, insbesondere die Belastbarkeit auf Zug oder Druck wird gemäss dem Stande der Technik für stiftförmige Implantate erhöht durch entsprechend ausgestaltete Gewinde (US-3499222), durch abspreizbare Elemente (z.B. US-5766009, EP-1184006) oder durch kragenförmige Elemente. Ankerförmige Implantate, die insbesondere für die Befestigung von Drähten und Nahtmaterialien verwendet werden, werden zur Erhöhung der primären und sekundären Stabilität für Zugbelastungen mit Widerhaken-ähnlichen Oberflächenstrukturen ausgestattet (US-4360343). Auch diese Verbesserungen erlauben keine Belastung der Implantate unmittelbar nach der Implantation.

Das Dokument EP0 358 601 offenbart eine Endoprothese mit einem Kern und einem Mantel aus einem thermoplastischen Werkstoff, wobei der Mantel den Kern umschließt.

Das Dokument WO 02/069817 A ist Stand der Technik nach Art. 54(3)EPÜ und offenbart ein Implantat mit einem zentralen Implantatteil sowie einem außen am zentralen Implantatteil angeordneten, peripheren Implantatteil, wobei zwischen dem zentralen Implantatteil und dem peripheren Implantatteil eine formschlüssige Verbindung vorhanden ist. Das zentrale Implantatteil weist Oberflächenbereiche einer ersten Art und vom peripheren Implantatteil gebildete, von den Oberflächenbereichen der ersten Art verschiedene Oberflächenbereiche einer zweiten Art auf. Die Oberflächenbereiche zweiter Art weist ein mittels mechanischer Schwingungen verflüssigbares Material auf, mit dessen Hilfe das Implantat bei einer Implantation mittels mechanischer Schwingungen im Knochengewebe mindestens primär stabilisierbar ist. Die Oberflächenbereiche erster Art sind für eine von einer primären Stabilisierung verschiedene, klinische Funktion ausgerüstet.

Die Erfindung stellt sich also die Aufgabe, ein in Knochengewebe oder durch Knochenersatzmaterial ergänztes Knochengewebe zu implantierendes Implantat zu schaffen, das eine sehr gute Primärstabilität hat, so dass es beispielsweise sofort nach der Implantation belastet werden kann, das aber zudem weitere, klinische Funktionen z.B. Förderung der Osseointegration, Durchlass von Partikeln oder Molekülen in der einen oder anderen Richtung (Freisetzung von therapeutischen Stoffen oder Drainage), elektrische oder chemische Stimulation etc., übernimmt und zwar ebenfalls unmittelbar nach der Implantation. Dabei sollen die weiteren, klinischen Funktionen durch die geforderte Primärstabilität klinisch nicht wesentlich eingeschränkt werden. Wenn das Implantat also beispielsweise eine lasttragende Funktion hat, also beispielsweise ein Dentalimplantat ist, soll es dank der verbesserten Primärstabilität sofort nach der Implantation oder mindestens bedeutend früher nach der Implantation als bekannte Implantate möglichst uneingeschränkt belastet werden können, wobei aber die Osseointegration (weitere, klinische Funktion) durch die Primärstabilität im wesentlichen unbeeinträchtigt bleibt, das heisst unmittelbar nach der Implantation beginnt, derart, dass die oben genannten positiven Effekte einer frühen Belastung auf die Osseointegration voll ausgenützt werden können. Weder das erfindungsgemässe Implantat noch dessen Implantation sollen aufwendiger sein als dies für Implantate gemäss dem Stande der Technik der Fall ist.

Diese Aufgabe wird gelöst durch das Implantat, wie es in den Patentansprüchen definiert ist.

Die Oberflächen des erfindungsgemässen Implantates, die nach der Implantation an das Knochengewebe angrenzen und beispielsweise vom Knochengewebe umwachsen oder mit diesem verwachsen sollen, weisen Bereiche einer ersten Art und Bereiche einer zweiten, von der ersten Art verschiedenen Art auf.

Die Oberflächenbereiche der ersten Art sind in an sich bekannter Weise für eine oder mehrere vorgegebene, klinische Funktionen ausgerüstet. Beispiele solcher vorgegebener klinischer Funktionen sind die Förderung oder mindestens Ermöglichung der Osseointegration für eine gute, sekundäre Stabilität, die Freisetzung von therapeutisch wirksamen Stoffen in das das Implantat umgebende Gewebe, die Entfernung von unerwünschten Stoffen (Drainage) aus dem das Implantat umgebende Gewebe oder die elektrische oder chemische Stimulation von das Implantat umgebendem Gewebe.

Die Oberflächenbereiche der ersten Art weisen also beispielsweise im Falle eines lasttragenden Implantates Strukturen auf, die sich für eine stabile Verwachsung oder Durchwachsung mit dem vitalen Gewebe eignen und sie sind mindestens bezüglich Osseointegration biologisch aktiv. Ferner oder zusätzlich dazu können durch die Oberflächenbereiche erster Art Stoffe mit beispielsweise osseointegrativer, entzündungshemmender, infektionsbekämpfender oder wachstumsfördernder Wirkung freigesetzt werden oder sie können für den Durchlass von therapeutisch wirkenden Stimulationsimpulsen ausgerüstet sein.

Die Oberflächenbereiche der ersten Art sind also beispielsweise biologisch verträgliche Oberflächen (z.B. aus Titan) und können makroskopische Strukturen bilden, in die das Knochengewebe einwachsen kann. Solche Oberflächen können zusätzlich beispielsweise mit Calziumphosphat-haltigen Verbindungen beschichtet sein, sie können beispielsweise mit Phosphonaten oder Peptidsequenzen modifiziert sein und/oder sie können Gele oder Polymere aufweisen, in denen beispielsweise Wachstumsfaktoren eingelagert sind.

Die Oberflächenbereiche der zweiten Art sind für die Erstellung der Primärstabilität ausgerüstet. Dafür weisen diese Oberflächenbereiche ein Material auf, das mittels mechanischer Schwingungen verflüssigbar ist, also beispielsweise ein sich thermoplastisch verhaltendes Material (thermoplastisches Material oder Verbundwerkstoff mit einer thermoplastischen Komponente) oder einen thixotropen Zement, wobei dieses verflüssigbare Material bei der Implantation mittels mechanischer Schwingungen, z.B. Ultraschall, verflüssigt und in Unebenheiten, Poren oder erstellten Geometrien im das Implantat umgebenden Knochengewebe eingepresst wird. Das die Oberflächenbereiche der zweiten Art bildende Material bildet bereits vor der Implantation Teile der Aussenoberfläche des Implantates, oder es befindet sich im Innern des Implantates und wird bei der Implantation in verflüssigtem Zustand durch entsprechende Öffnungen an die Aussenoberfläche des Implantates gepresst, wo es dann in situ die Oberflächenbereiche der zweiten Art bildet.

Damit das verflüssigte Material der Oberflächenbereiche der zweiten Art bei der Implantation in das Knochengewebe eingepresst werden kann, sind die Oberflächenbereiche der zweiten Art derart angeordnet, dass sie bei der Positionierung des Implantates im Knochen mit dem Knochengewebe in Berührung kommen. Das heisst, die Oberflächenbereiche der zweiten Art überragen beispielsweise mindestens örtlich die Oberflächenbereiche der ersten Art oder sie befinden sich an Implantatskanten, Ausbuchtungen etc. An Implantaten, die nicht Teil der Erfindung sind und die das die Oberflächenbereiche der ersten Art bildende Material vor der Implantation in ihrem Inneren tragen, sind die Öffnungen, durch die das verflüssigbare Material ausgepresst wird, vorteilhafterweise an ebensolchen Stellen angeordnet.

Die Oberflächenbereiche der beiden verschiedenen Arten sind derart angeordnet und das verflüssigbare Material und/oder die Verflüssigung werden derart dosiert angewendet, dass die Oberflächenbereiche der ersten Art von verflüssigtem Material möglichst frei bleiben. Damit wird erreicht, dass die weiteren, klinischen Funktionen dieser Oberflächenbereiche der ersten Art auch unmittelbar nach der Implantation nicht oder höchstens in einem klinisch nicht relevanten Masse behindert werden. Dadurch wird also beispielsweise erreicht, dass die Entstehung der sekundären Stabilität durch Osseointegration in Oberflächenbereichen der ersten Art nicht nur nicht behindert sondern auch nicht verzögert wird, so dass diese unmittelbar nach der Implantation beginnen kann.

Die Trennung der beiden Arten von Implantatsoberflächen wird erfindungsgemäß für Implantate, die während der Implantation relativ zum Knochengewebe in einer Implantationsrichtung bewegt werden, erreicht, indem die beiden Arten von Oberflächenbereichen in Implantationsrichtung im wesentlichen parallel nebeneinander angeordnet werden.

Das erfindungsgemässe Implantat wird wie bekannte Implantate in einer spezifisch für das Implantat im gegebenenfalls vorgängig regenerierten Knochengewebe (z.B. des Kiefers) erstellten Öffnung implantiert, wobei diese Öffnung das ganze Implantat (Wurzelbereich) aufnehmen kann oder wobei das Implantat selbstschneidend tiefer als die Öffnung in das Knochengewebe eingebracht werden kann. Die Öffnung kann also beispielsweise nur die Kortikalisschicht betreffen oder bei entsprechender Ausgestaltung des Implantates kann sie überhaupt fehlen. Das erfindungsgemässe Implantat kann auch im Sinne einer Replika in seiner Form an eine unregelmässige Öffnung im Knochengewebe angepasst sein, also beispielsweise die Form einer entfernten, natürlichen Zahnwurzel haben und direkt in diese unregelmässige Öffnung implantiert werden.

Das erfindungsgemässe Implantat ist also beispielsweise ein Dentalimplantat und hat die Form eines Stifts oder einer natürlichen Zahnwurzel und weist an seinem proximalen Ende eine Fixierstelle (z.B. Sackloch mit Innengewinde oder Stelle, an der der Dentalchirurg ein solches Sackloch erstellen kann) oder einen künstlichen Kronenbereich auf. Es kann an seinem distalen Ende meisselförmig ausgebildet sein und/oder seitlich mit selbstschneidenden oder furchenden Strukturen ausgerüstet sein. Es kann ferner platten-, scheiben- oder klingenförmig sein und eine oder mehrere Fixierstellen aufweisen oder es kann die Form eines Ankers haben, an dem beispielsweise ein Draht oder ein Nahtmaterial befestigbar ist.

Das erfindungsgemässe Implantat ist einteilig und weist die oben definierten, verschiedenen Oberflächenbereiche auf, die beispielsweise aus verschiedenen Materialien bestehen. Bei einer Ausführungsform, welche nicht Teil der Erfindung ist, weist das Implantat das verflüssigbare Material in seinem Innern auf, wobei Öffnungen vorgesehen sind, durch die das Material in seinem verflüssigten Zustand an die Aussenseite des Implantates gepresst werden kann. Das Implantat kann auch zwei- oder mehrteilig sein, wobei der Chirurg zwei oder mehrere, aus verschiedenen Materialien bestehende Teile zu einem Implantat kombiniert.

Für die Implantation wird das erfindungsgemässe Implantat in einer Öffnung in einem Knochen (oder mit Knochenersatzmaterial ergänztem Knochengewebe), beispielsweise in einem Kieferknochen, oder gegebenenfalls auf dem Knochen positioniert und dann mit mechanischen Schwingungen, beispielsweise mit Ultraschall beaufschlagt und gleichzeitig gegen den Knochen gepresst. Dadurch wird mindestens ein Teil des verflüssigbaren Materials verflüssigt und in Poren, Oberflächenunebenheiten oder geschaffene Geometrien des umliegenden Knochengewebes gepresst, wo es nach der Erstarrung eine formschlüssige Verbindung zwischen Implantat und umliegendem Knochengewebe und gegebenenfalls Knochenersatzmaterial bildet. Je nach Ausführungsform des Implantates wird es gleichzeitig mit der Verflüssigung auch im Knochengewebe vorgetrieben (Implantationsrichtung).

Zur Beaufschlagung des positionierten Implantates mit mechanischen Schwingungen wird auf dessen proximalem Ende beispielsweise die Sonotrode eines Ultraschallgerätes aufgesetzt. Experimente zeigen, dass mit einer Leistung von 0,2 bis 20 W pro Quadratmillimeter Wirkfläche gute Resultate erzielt werden. Die Frequenz der Schwingungen beträgt zwischen 2 und 200 kHz.

Erfindungsgemässe Implantate mit einer lasttragenden Funktion (z.B. Dentalimplantate) weisen beispielsweise einen zentralen Implantatteil auf, der die Oberflächenbereiche der ersten Art trägt und der beispielsweise aus Metall (z.B. Stahl, Titan, Cobalt/Chrom-Legierungen), aus keramischem oder glasartigen Material (z.B. Aluminiumoxid, Zirkonoxid, Silikate, Calziumphosphat-Keramiken oder -Gläser), aus duroplastischen oder hochtemperatur-thermoplastischen Kunststoffen (Polyetherarylketone, Polyfluor- oder Polychlorethylene, Polyetherimide, Polyethersulfone, Polyvinylchlorid, Polyurethane, Polysulfone, Polyester) oder aus einem Verbundwerkstoff (z.B. kohlefaserverstärkter Hochtemperatur-Thermoplast) besteht sowie einen peripheren Implantatteil aus dem verflüssigbaren Material, beispielsweise aus einem Material mit thermoplatischen Eigenschaften. Bei einer Ausführungsform, die nicht Teil der Erfindung ist, kann das verflüssigbare Material im Innern eines hohlen, zentralen Implantatteils vorgelegt sein, wobei die Wandung des zentralen Implantatteils durchgehende Öffnungen aufweist, durch die das verflüssigte Material unter dem Einfluss der mechanischen Schwingungen gepresst wird, um aussen Oberflächenbereiche der zweiten Art zu bilden. Die Implantatteile können herstellerseitig miteinander verbunden werden oder erst unmittelbar vor oder bei der Implantation durch den Chirurgen miteinander in Verbindung gebracht werden.

Erfindungsgemässe Implantate ohne nennenswerte, lasttragende Funktion (z.B. Implantate mit einer Freisetzungs-, Drainage- oder Stimulationsfunktion) können ebenfalls einen zentralen Implantatteil und einen peripheren Implantatteil aus einem mindestens teilweise verflüssigbaren Material aufweisen, wobei die mechanische Stabilität (tragende Funktion), die für die Implantation notwendig ist, durch den peripheren Teil übernommen werden kann und der zentrale Teil in einem solchen Fall eine nur geringe mechanische Stabilität aufweist. Ein solcher zentraler Implantatteil kann beispielsweise ein durchlässiger Behälter beispielsweise aus porösem Calziumphosphat oder anderen mechanisch wenig stabilen Knochenersatzmaterial oder aus einer dünnen Membran sein, wobei die Freisetzung bzw. Drainage oder Stimulation durch die Behälterwandung stattfindet. Der zentrale Implantatteil kann auch ein Körper aus beispielsweise porösem Calziumphosphat oder anderem Knochenersatzmaterial sein, dessen Funktion darin besteht, die Bildung von fehlendem oder zusätzlich erwünschtem Knochenmaterial zu initiieren oder zu unterstützen. Auch im Falle eines mechanisch wenig stabilen, zentralen Implantatteils ist es möglich, bei einer Ausführungsform, die nicht Teil der Erfindung ist, das verflüssigbare Material im Innern des zentralen Implantatteils vorzusehen und während der Implantation in verflüssigtem Zustand durch entsprechende Öffnungen des zentralen Implantatteiles auf dessen Aussenseite zu pressen.

Das erfindungsgemässe Implantat kann auch nur aus einem einzigen Material bestehen, wenn dieses die Anforderungen der mechanischen Festigkeit des Implantates und gegebenenfalls einer Fixierstelle, die durch die weiteren klinischen Funktionen der Oberflächenbereiche erster Art (z.B. biologische Integration bzw. sekundäre Stabilität) gestellten Anforderungen und die Anforderungen der Verflüssigbarkeit durch mechanische Schwingungen erfüllen kann. Gegebenenfalls kann das einzige Material in verschiedenen Bereichen des Implantates zu verschiedenen Graden gefüllt sein (z.B. mit Fasern, Whiskers oder Partikeln) oder es kann in verschiedenen Bereichen mit verschiedenen Materialien gefüllt sein. Auch in diesem Falle ist durch entsprechende Ausgestaltung der im Knochengewebe zu integrierenden Oberflächenbereiche dafür zu sorgen, dass die Oberflächenbereiche der zweiten Art bzw. das verflüssigte Material bei der Implantation insbesondere mit dem Knochengewebe in Berührung kommen und dass das verflüssigte Material nicht oder nur zu einem klinisch nicht relevanten Grad auf die Oberflächenbereiche der ersten Art getragen werden.

Das verflüssigbare Material ist im Falle von Implantaten, deren Oberflächenbereiche der ersten Art für eine Osseointegration ausgerüstet sind, vorteilhafterweise mindestens teilweise biologisch abbaubar (resorbierbar), so dass die durch den Formschluss zwischen dem Implantat und dem Knochengewebe erstellte Primärstabilität allmählich abgelöst wird durch die Sekundärstabilität der Osseointegration, die vorteilhafterweise in demselben Masse zunimmt, wie das verflüssigbare Material resorbiert, das heisst die primäre Stabilität reduziert wird. Insbesondere im Falle von osteoporotischem Knochengewebe oder schlecht regenerierendem Knochengewebe mag es auch vorteilhaft sein, die primäre Stabilisierung als Ergänzung zur sekundären Stabilisierung dauernd zu erhalten, das heisst, ein nicht resorbierbares, verflüssigbares Material einzusetzen, wobei dieses gegebenenfalls auch selbst für eine gute biologische Integration (sekundäre Osseointegration) ausgerüstet sein kann.

In Implantaten mit anderen, klinischen Funktionen als lasttragenden Funktionen ist das verflüssigbare Material vorteilhafterweise mindestens teilweise resorbierbar, wenn das Implantat nach einer vorgegebenen Zeit entfernt werden soll oder vollständig durch Knochengewebe ersetzt werden soll. Wenn die primäre Stabilität aufrechterhalten bleiben soll, ist das verflüssigbare Material nicht oder nur teilweise resorbierbar.

Als verflüssigbare Materialien eignen sich zum Beispiel resorbierbare Polymere beispielsweise auf Milch- und/oder Glykolsäurebasis (PLA, PLLA, PGA, PLGA etc.) oder Polyhydroxyalkanoate (PHA), Polycaprolactone (PCL), Polysacharide, Polydioxanone (PD) Polyanhydride, Polypeptide oder entsprechende Copolymere oder Mischpolymere oder die genannten Polymere als Komponente enthaltende Verbundwerkstoffe. Als nicht resorbierbare Polymere sind Thermoplasten wie beispielsweise Polyolefine (z.B. Polyethylen), Polyacrylate, Polymetacrylate, Polycarbonate, Polyamide, Polyester, Polyurethane, Polysulfone, Polyarylketone, Polyimide, Polyphenylsulfide oder Flüssig-Kristall-Polymere (liquid cristal polymers LCPs), Polyacetale, halogenierte Polymere, insbesondere halogenierte Polyolefine, Polyphenylensulfide, Polysulfone, Polyether oder entsprechende Copolymere und Mischpolymere oder die genannten Polymere als Komponente enthaltende Verbundwerkstoffe geeignet. Anwendbare thixotrope Systeme sind resorbierbare, teilresorbierbare oder nicht resorbierbare, polymere, keramische oder hydraulische Zemente (z.B. Norian® von Synthes oder Sulfix® von Centerpulse).

Das verflüssigbare Material kann für weitere Funktionen Fremdphasen oder weitere Stoffe enthalten. Insbesondere kann das verflüssigbare Material durch Beimischen von Fasern oder Whiskern (z.B. Calziumphosphat-Keramiken oder -Gläser) verstärkt sein (Verbundwerkstoff). Es kann auch in situ quellende oder lösliche (porenbildende) Bestandteile (z.B. Polyester, Polysaccharide, Hydrogele, Natriumphosphat) oder in situ freizusetzende Stoffe mit therapeutischer Wirkung beispielsweise zur Förderung von Heilung und Regeneration (z.B. Wachstumsfaktoren, Antibiotika, Entzündungshemmer oder Puffer wie Natriumphosphat gegen nachteilige Wirkungen sauren Abbaus) enthalten. Wenn das verflüssigbare Material resorbierbar ist, werden solche Stoffe verzögert freigesetzt.

Der Implantatteil, der nicht aus dem verflüssigbaren Material besteht, ist nicht resorbierbar, wenn das Implantat im Körper verbleiben oder chirurgisch entfernt werden soll. Er kann aber auch mindestens teilweise aus einem resorbierbaren Material bestehen, das nach der Implantation allmählich durch vitales Gewebe ersetzt wird.

Die Ausgestaltung des Implantates und die Wahl des verflüssigbaren Materials sind aufeinander abzustimmen, derart, dass die Festigkeit des Formschlusses der erwarteten Belastung genügen kann, und derart, dass die Verflüssigung eine verantwortbare, das heisst möglichst geringe Freisetzung von Wärme mit sich bringt. Wenn verflüssigbare Materialien mit einer relativ hohen Erweichungstemperatur verwendet werden, ist vorteilhafterweise dafür zu sorgen, dass das Implantat als Ganzes (inklusive verflüssigbares Material) die mechanischen Schwingungen im Sinne eines Resonators leitet, so dass das verflüssigbare Material nur sehr örtlich, z.B. nur im Bereiche von entsprechend vorgesehenen Energierichtungsgebern in den Oberflächenbereichen der zweiten Art verflüssigt wird. Auf diese Weise kann die freigesetzte Wärmemenge in einem akzeptablen Rahmen gehalten werden. Insbesondere bei Verwendung eines Materials mit einer relativ tiefen Erweichungstemperatur oder eines ohne Freisetzung von Wärme verflüssigbaren Materials (z.B. thixotrope Zemente) kann die Verflüssigung auch im Innern des verflüssigbaren Materials (durch starke Dämpfung der anregenden Schwingungen) oder an Berührungsstellen zwischen zentralem und peripherem Implantatteil erfolgen.

Die Wärmebelastung des Gewebes während der Implantation kann weiter reduziert werden, indem der zentrale Implantatteil Materialien mit einer hohen Wärmeleitfähigkeit und/oder einer hohen Wärmekapazität (z.B. Siliziumcarbid) aufweist und gegebenenfalls mit Kühlkanälen versehen ist, durch die ein Kühlmedium geleitet wird.

Implantate werden anhand der folgenden Figuren detailliert beschrieben. Dabei zeigen:
- **Figuren 1, 2A, 2B und 2C**: drei erste, beispielhafte Ausführungsformen eines im wesentlichen stiftförmigen, erfindungsgemässen Implantates (z.B. Dentalimplantat) mit einem zentralen und einem peripheren Implantatteil als Seitenansicht (Figur 1) und im Querschnitt (Figuren 2A bis 2C);
- **Figur 3**: eine zweite, beispielhafte Ausführungsform eines erfindungsgemässen Implantates (z.B. Dentalimplantat) mit einem zentralen und einem peripheren Implantatteil, wobei die Form des Implantates an eine bestehende Kavität in einem Knochen (z.B. durch Entfernung einer natürlichen Zahnwurzel entstandene Öffnung im Kieferknochen) angepasst ist;
- **Figuren 4 und 5**: zwei weitere Implantate (z.B. Dentalimplantat) mit zentralem und peripherem Implantatteil, wobei der zentrale Implantatteil an eine bestehende Kavität in einem Knochen angepasst (z.B. Nachbildung einer natürlichen Zahnwurzel) ist und selbstscheidend oder furchend ausgerüstet ist (Querschnitt);
- **Figur 6**: ein Implantat (z.B. Dentalimplantat) mit einem zentralen und einem peripheren Implantatteil (Seitenansicht);
- **Figuren 7 und 8**: eine beispielhafte Ausführungsform eines erfindungsgemässen Implantates in der Form eines Ankers als Seitenansicht (Figur 7) und im Querschnitt (Figur 8);
- **Figuren 9 und 10**: eine beispielhafte Ausführungsform eines platten-, scheiben- oder klingenförmigen erfindungsgemässen Implantates (z.B. Dentalimplantat mit zwei Fixierstellen) als Seitenansicht (Figur 9) und Draufsicht (Figur 10);
- **Figuren 11 und 12**: ein im wesentlichen stiftförmiges Implantat (z.B. Dentalimplantat) mit einem hohlen zentralen Implantatteil als Längsschnitt (Figur 11) und Draufsicht (Figur 12).
- **Figur 13**: eine beispielhafte Ausführungsform des erfindungsgemässen Implantates mit einem zentralen Implantatteil ohne wesentliche mechanische Stabilität;
- **Figur 14**: ein Augmentationselement als Implantat;

- **Figuren 15 und 16 (je A, B und C)**: zwei Implantate zur Verbindung von zwei Rückenwirbeln je dreidimensional dargestellt (Figuren 15A und 16A), während der Implantation zwischen zwei Wirbelkörpern als Seitenansicht (Figuren 15B und 16B) und in implantiertem Zustand als Frontansicht (Figuren 15C und 16C).

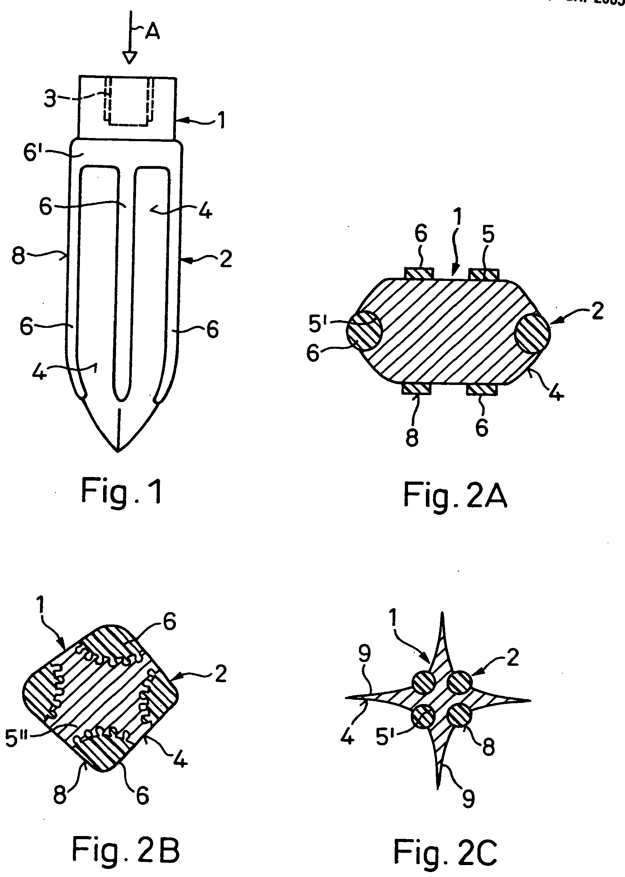

**Figuren 1 und 2A bis 2C** zeigen eine beispielhafte, stiftförmige Ausführungsform des erfindungsgemässen Implantates, das eine lasttragende Funktion hat, das also beispielsweise ein Dentalimplantat ist aber auch ein Implantat zur orthopädischen Verwendung, beispielsweise zur Stabilisierung eines Knochenbruches oder zur Fixierung einer Platte oder ein Schaft einer Gelenkprothese (z.B. Hüftgelenk, Kniegelenk, Schultergelenk oder Fingergelenk) sein kann. Das Implantat weist einen zentralen Implantatteil 1 und einen peripheren Implantatteil 2 auf, wobei der zentrale Implantatteil an seinem proximalen Ende beispielsweise eine Fixierstelle 3, z.B. ein Sackloch mit Innengewinde oder eine Stelle, an der der Chirurg (z.B. Dentalchirurg) ein derartiges Sackloch erstellen kann, aufweist. Das distale Ende ist beispielsweise für eine selbstschneidende Wirkung meisselförmig ausgerüstet. Das Implantat kann auch, wie durch den Querschnitt gemäss Figur 2C veranschaulicht, beispielsweise axial verlaufende, selbstschneidende oder furchende Elemente 9 aufweisen. Der zentrale Implantatteil 1 weist sich parallel zur Implantationsrichtung A erstreckende Oberflächenbereiche 4 der ersten Art (z.B. mit osseointegrativen, entzündungshemmenden, infektionsbekämpfenden und/oder wachstumsfördernden Eigenschaften) auf und zwischen den Oberflächenbereichen 4 der ersten Art Oberflächen, die sich für eine Verbindung mit dem peripheren Implantatteil 2 eignen. Die Verbindung zwischen dem peripheren Implantatteil 2 und dem zentralen Implantatteil kann eine adhesive Verbindung 5 (Figur 2A) sein, ist aber erfindungsgemäß eine formschlüssige, beispielsweise einzelne Nuten 5' (Figuren 2A und 2C) mit verengtem Öffnungsschlitz oder Oberflächen 5" mit einer Vielzahl von Öffnungen oder Nuten (Figur 2B). Der periphere Implantatteil 2 weist Finger 6 auf, die beispielsweise in die Nuten 5' oder auf die Oberflächenbereiche 5" passen und die mindestens einen Teil der Oberflächenbereiche 8 der zweiten Art bilden.

Wie aus den Figuren 2A bis 2C hervorgeht, stellt die Erfindung an den Querschnitt der erfindungsgemässen, stiftförmigen Implantate eigentlich keine Bedingungen, so dass dieser funktionsabhängig wählbar ist. Es sind also auch andere Querschnitte als in den drei Figuren dargestellt denkbar, zum Beispiel ein zentraler Implantatteil mit einem runden Querschnitt und darauf sitzende Finger 6, wie sie in der Figur 2A dargestellt sind.

Insbesondere das in der Figur 2C illustrierte Implantat kann beispielsweise weitgehend selbstschneidend in das Knochengewebe getrieben werden. Damit dabei das verflüssigte Material nicht auf die Oberflächenbereiche 4 der ersten Art getrieben wird, erstrecken sich die Oberflächenbereiche erster und zweiter Art (4 und 8) nebeneinander parallel zur Implantationsrichtung A. Im proximalen Bereich, wo der Implantationsweg nur noch kurz ist, können die Finger 6 in einen Ring 6' münden, der sich um den zentralen Implantatteil 1 erstreckt und vorteilhafterweise ebenfalls in einer Nut des zentralen Implantatteils 1 festgehalten ist. Durch den Ring 6' werden nicht nur die Finger 6 zu einem zusammenhängenden, peripheren Implantatteil 2 zusammengefasst, was für eine Verbindung mit dem zentralen Implantatteil gegebenenfalls durch den Chirurgen von Vorteil ist, sondern es wird auch, gegebenenfalls im Bereich der Kortikalis eine innigere primäre Stabilisierung zwischen Implantat und Knochengewebe insbesondere gegen Zug und Torsion geschaffen. Gegebenenfalls ist in der Kortikalis ein Gewinde oder eine ähnliche Struktur zu erstellen, damit sich der Ring 6' formschlüssig mit dieser relativ dichten Knochenschicht verbinden kann.

Für ein Implantat, das in einer tieferen Öffnung positioniert und während der Beaufschlagung mit den mechanischen Schwingungen nicht oder nur wenig vorgeschoben wird, können die Oberflächenbereiche erster und zweiter Art auch anders angeordnet sein. Die Oberflächenbereiche 8 der zweiten Art können anstelle von Fingern 6 beispielsweise ein Muster von Punkten oder von sich kreuzenden Linien bilden. Die Anordnung der Oberflächenbereiche 8 der zweiten Art ist also an die Implantationsart anzupassen. Zusätzlich ist sie an die Primärstabilität anzupassen, die durch das verflüssigte Material zu erreichen ist, die also nicht durch die Formgebung des Implantates gegeben werden kann.

Die beiden Implantatteile 1 und 2 der in den Figuren 1 und 2A bis 2C dargestellten Implantate können herstellerseitig miteinander verbunden werden. Der periphere Implantatteil 2 kann beispielsweise durch Spritzgiessen direkt auf dem zentralen Implantatteil 1 hergestellt werden. Die beiden Implantatteile 1 und 2 können auch separat hergestellt werden und erst unmittelbar vor der Implantation vom Chirurgen zusammengefügt werden. Dabei ist es vorteilhaft, die erfindungsgemäße formschlüssige oder beispielsweise adhesive Verbindung zwischen den beiden Materialien während der Implantation zu erzielen, dadurch, dass das Material des peripheren Implantatteils 2 verflüssigt und beispielsweise in die Öffnungen oder Nuten gemäss Figur 2B des zentralen Implantatteils gepresst wird. Dafür ist gegebenenfalls die Innenseite des peripheren Implantatteils 2 oder die entsprechende Oberfläche des zentralen Implantatteils 1 mit Energierichtungsgebern zu versehen.

Der Vorteil eines verbraucherseitigen Zusammenfügens besteht darin, dass die beiden Teile separat und dadurch gegebenenfalls in verschiedenen Verfahren, die an die verschiedenen Funktionalitäten der Teile angepasst sind, sterilisiert werden können. Eine Sterilisation des zusammengefügten Implantates entfällt. Das verbraucherseitige Zusammenfügen des Implantates erlaubt es auch, dem Chirurgen ein Set zur Verfügung zu stellen mit sich voneinander beispielsweise bezüglich Länge und Durchmesser unterscheidenden zentralen Implantatteilen und sich voneinander beispielsweise bezüglich Material oder Fingerdicke unterscheidenden peripheren Implantatteilen, so dass der Chirurg ein genau für einen vorliegenden Fall geeignetes Implantat selber zusammenstellen kann (grössere Variabilität mit einer kleineren Zahl von Systemkomponenten).

Zur Implantation der stiftförmigen Implantate gemäss Figuren 1 und 2A bis 2C wird eine Vorrichtung (z.B. Sonotrode einer Ultraschallvorrichtung) verwendet, deren distales Ende im wesentlichen auf die proximale Stirnfläche des Implantates abgestimmt ist. Gegebenenfalls wird zwischen Sonotrode und Implantat eine Kopplungselement eingefügt. Die Schwingungsenergie wird vorteilhafterweise über den zentralen Implantatteil eingekoppelt.

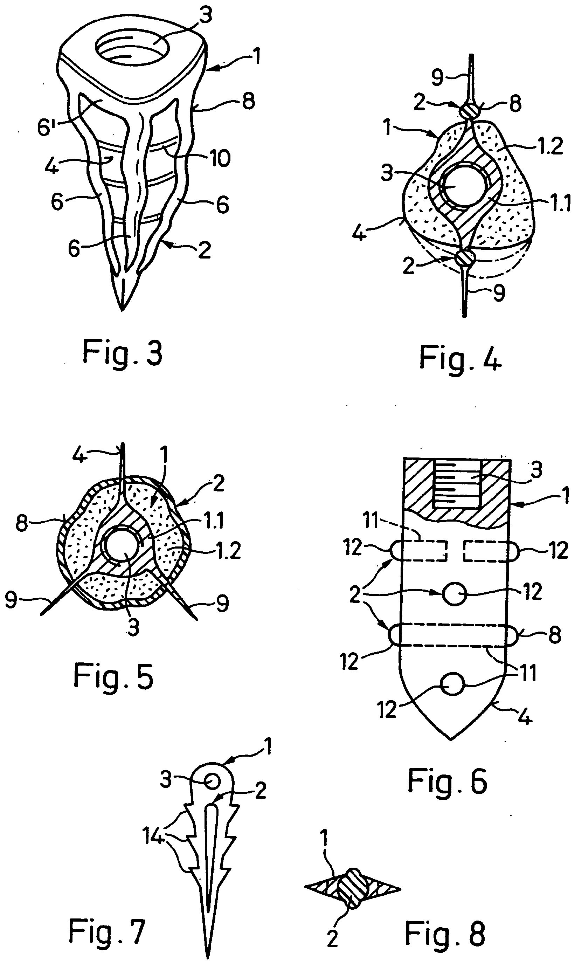

**Figur 3** zeigt ein erfindungsgemässes Dentalimplantat, das im Prinzip wie das Implantat gemäss Figur 1 ausgestaltet ist, das aber in seiner Form nicht den bekannten stift- oder schraubenförmigen Implantaten nachgebildet ist, sondern in seiner Form an eine bestehende Kavität in einem Knochen, im vorliegenden Fall an eine natürliche Zahnwurzel angepasst ist. Der zentrale Implantatteil 1 ist zwischen den Oberflächenbereichen 8 der zweiten Art, die durch den peripheren Implantatteil 2 gebildet werden, also in den Oberflächenbereichen 4 der ersten Art mit Strukturen 10 versehen, die wie ein Gewinde eine bessere Verankerung im regenerierten Knochengewebe (sekundäre Stabilität) ermöglicht.

**Figuren 4 und 5** zeigen im Querschnitt zwei weitere Implantate, die sich für die Implantation in eine bestehende Kavität in einem Knochen, beispielsweise in der durch Entfernung einer natürlichen Zahnwurzel eignen. Sie sind für diese Anwendung an die entsprechende Kavität angepasst und weisen axial verlaufende selbstschneidende oder furchende Elemente 9 auf. Der zentrale Implantatteil 1 der beiden Implantate besteht aus einem Stiftteil 1.1, der beispielwsweise eine Fixierstelle 3 oder eine künstlicher Zahnkrone trägt, und aus einem Formteil 1.2. Der Formteil 1.2 wird ex situ im Sinne einer Replika beispielsweise anhand einer aus einem Kiefer entfernten Zahnwurzel, wie dies beispielsweise in der Publikation US-6132214 (Suhonen et al.) beschrieben ist, oder in situ, d.h. in der entsprechenden Kavität geformt.

Der Formteil 1.2 gemäss Figur 4 bildet die Oberflächenbereiche 4 der ersten Art (z.B. mit osseointegrativen, entzündungshemmenden, infektionsbekämpfenden und/oder wachstumsfördernden Eigenschaften) und besteht aus einem vorteilhafterweise resorbierbaren oder teilweise resorbierbaren Knochenersatzmaterial (z.B. Calziumphosphat, Polylactid, mit Calziumphosphat gefülltes, nicht resorbierbares Polymer, Verbindungssystem mit verstärkenden Elementen). Der periphere Implantatteil 2 beschränkt sich auf die selbstschneidenden oder furchenden Elemente 9, in die beispielsweise stiftförmige Teile aus dem verflüssigbaren Material eingelassen sind.

Das Implantat gemäss Figur 4 kann auch in zwei Schritten implantiert werden. Dabei wird zuerst eine vorhandene Kavität mit einem Stück Knochenersatzmaterial (Formteil 1.2) gefüllt und wird dann der Stiftteil 1.1 eingesetzt, wobei die Verankerung mittels verflüssigbarem Material (peripherer Implantateil 2) mindestens teilweise das Knochenersatzmaterial betreffen kann. Solche Fälle sind in der Figur 4 mit strichpunktierten Linien angedeutet.

Der Formteil 1.2 gemäss Figur 5, welche nicht Teil der Erfindung ist, ist von einer relativ dünnen und möglichst flexiblen Schicht des verflüssigbaren Materials, also vom peripheren Implantatteil 2 umgeben, der die Oberfläche 8 der zweiten Art bildet. Anstelle der dünnen Schicht kann auch eine mindestens teilweise mit dem verflüssigbaren Material beschichtete Membran vorgesehen werden. Die axial verlaufenden, selbstschneidenden oder furchenden Elemente 9 weisen die Oberflächen 4 der ersten Art auf. Der Formteil 1.2 besteht aus einem plastischen, aushärtbaren Material, beispielsweise aus einem mittels Licht, Ultraschall oder Wärme aushärtbaren oder hydraulischen Knochenzement, der vorteilhafterweise thixotrope Eigenschaften hat. Beim Einbringen in die Knochenkavität passt sich der Formteil 1.2 an diese Kavität an. Beim Beaufschlagen mit mechanischen Schwingungen wird nicht nur das verflüssigbare Material der Oberflächenbereiche 8 der zweiten Art in Poren und Unebenheiten des umliegenden Knochengewebes eingepresst sondern auch der Formkörper an die Öffnung im Kieferknochen angepasst und gegebenenfalls ausgehärtet. Das verflüssigbare Material ist vorteilhafterweise resorbierbar, so dass die durch die an den Oberflächenbereichen 8 der zweiten Art erstellte Primärstabilität abgelöst wird durch eine Sekundärstabilität, die zuerst durch die Osseointegration des Formkörpers 1.2 und nach dessen Resorption durch eine Osseointegration des Stiftteils 1.1 bedingt ist.

Als Dentalimplantate ausgebildete Implantate gemäss Figuren 4 und 5 können im wesentlichen unmittelbar nach Entfernung einer natürlichen Zahnwurzel im Kieferknochen implantiert werden, da ihre Form an die durch die Entfernung entstehende Öffnung anpassbar ist. Dank der durch die Oberflächenbereiche 8 der zweiten Art erzielten Primärstabilität können sie auch sofort belastet werden, wodurch Mikrobewegungen mit physiologischen Ausmassen entstehen, die die Osseointegration in den Oberflächenbereichen der ersten Art beschleunigen. Solche Dentalimplantate verkürzen die Behandlungszeit also noch mehr als die Implantate gemäss Figuren 1 bis 3. Dasselbe gilt selbstverständlich auch für Implantate, die in einer vorgegebenen Kavität von anderen als Kieferknochen zu implantieren sind.

**Figur 6** zeigt ein stiftförmiges Implantat, welches nicht Teil der Erfindung ist, (z.B. Dentalimplantat, Fixierung von Knochenbrüchen, Befestigung von Stabilisierungsplatten, Schaft von Gelenkprothesen) mit einem zentralen Implantatteil und einem peripheren Implantatteil 2. Der zentrale Implantatteil 1 weist durchgehende und/oder nicht durchgehende Öffnungen 11 zur Durchwachsung mit Knochengewebe auf, in denen, beispielsweise durch Reibschluss festgehalten, Stifte 12 aus dem verflüssigbaren Material stecken und über die Oberfläche des zentralen Implantatteils 1 vorstehen. Die Stifte 12 bilden zusammen den peripheren Implantatteil 2, die aus den Öffnungen 11 vorstehenden Stiftenden die Oberflächen 8 der zweiten Art.

**Figuren 7 und 8** zeigen als Seitenansicht und im Querschnitt eine ankerförmige Ausführungsform des erfndungsgemässen Implantates, dessen Fixierstelle 3 beispielsweise als Öse ausgebildet ist. Der Anker hat eine an sich bekannte Form und weist einen über seine Länge verlaufenden Schlitz auf, in dem formschlüssig ein Stift aus dem verflüssigbaren Material (peripherer Implantatteil 2) angeordnet ist. Der Stift 13 steht beidseitig über die Ankeroberfläche vor. Das ankerförmige Implantat kann wie bekannte solche Implantate zusätzlich Widerhaken 14 aufweisen, die bei einer Belastung auf Zug in das Knochengewebe gepresst werden und die die formschlüssige Verankerung durch den peripheren Implantatteil 2 ergänzen. Solche Widerhaken oder ähnliche Mittel können aber auch vollständig fehlen.

Die Ausgestaltung der Ankerkanten als Schneidklingen erleichtert die Implantation ohne vorgängige Erstellung einer entsprechenden Öffnung im Knochengewebe oder mit Erstellung einer Öffnung, die nur die Kortikalis betrifft.

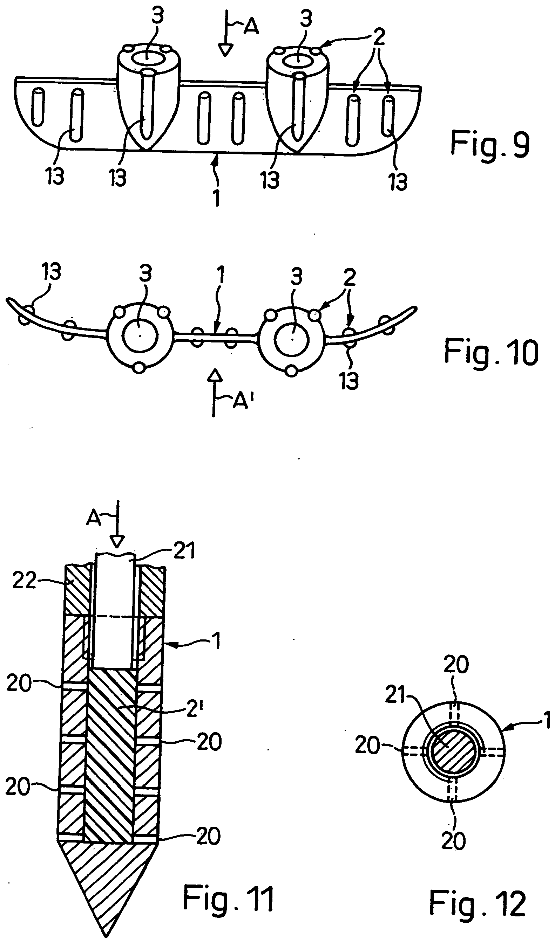

**Figuren 9 und 10** zeigen als weitere, beispielhafte Ausführungsform des erfindungsgemässen Implantates ein platten-, scheiben- oder klingenförmiges Dentalimplantat, das beispielsweise zwei Fixierstellen 3 oder zwei künstliche Zahnkronen aufweist und dessen peripherer Implantatteil 2 aus einer Mehrzahl von stiftförmigen Teilen 13 besteht, die im Bereiche der Platte, Scheibe oder Klinge in durchgehenden Öffnungen und im Bereiche der Fixierstellen in Nuten des zentralen Implantatteils angeordnet sind.

Die platten-, scheiben- oder klingenförmigen Dentalimplantate, von denen ein Beispiel in den Figuren 9 und 10 dargestellt ist, werden üblicherweise wie die stiftförmigen Dentalimplantate vom Kieferkamm her in den Kiefer eingeführt oder während der Beaufschlagung mit den mechanischen Schwingungen im Kiefer vorgetrieben (Implantationsrichtung A, Figur 9). Gegebenenfalls können sie auch von der Seite in den Kieferknochen implantiert werden (Implantationsrichtung A', Figur 10), wofür ein Teil des Kieferknochens zu entfernen und nach der Implantation wieder zu positionieren ist.

Platten-, scheiben- oder klingenförmige Implantate finden nicht nur im Dentalbereich Anwendung sondern bekannterweise auch im orthopädischen Bereich, wobei ihr proximaler Bereich dann entsprechend ausgestaltet ist.

**Figuren 11 und 12** zeigen ein weiteres stiftförmiges Implantat, welches nicht Teil der Erfindung ist, (z.B. Dentalimplantat oder Implantat für orthopädische Anwendung) im Längsschnitt und als Draufsicht. Der zentrale Implantatteil 1 ist als Hülse mit Innenhohlraum 2' ausgestaltet, in der das verflüssigbare Material vorgelegt ist. Die Hülsenwand weist durchgehende Öffnungen oder Schlitze 20 auf, die beispielsweise in axialen Reihen angeordnet sind oder sich axial erstrecken. Wenn das Implantat in einer Öffnung im Knochengewebe positioniert ist, wird ein schwingendes Element 21 (Sonotrode eines Ultraschallgerätes) auf das verflüssigbare Material im Innenhohlraum des zentralen Implantatteils aufgesetzt und dieses Material mit Schwingungen beaufschlagt, während es in Richtung des distalen Implantatsendes gepresst wird. Durch die Schwingungen wird das Material verflüssigt und durch den Druck durch die Öffnungen oder Schlitze 20 und in Oberflächenunebenheiten und Poren des umgebenden Knochengewebes gepresst, wodurch der das Implantat primär stabilisierende Formschluss entsteht.

Wenn sein zentraler Implantatteil 1 wie dargestellt mit einem meisselförmigen, distalen Ende versehen ist, kann auch das Implantat gemäss Figuren 11 und 12 ohne Öffnung in das Knochengewebe (mindestens Spongiosa) eingetrieben werden. Dafür eignet sich eine ringförmige Sonotrode 22. Sobald das Implantat die vorgegebene Position im Kieferknochen erreicht hat, kommt die Sonotrode 21 zum Einsatz.

In einem Implantat gemäss Figuren 11 und 12 wird also der periphere Implantatteil eigentlich erst nach der Positionierung des Implantates im Knochengewebe, also in situ erstellt.

Das im Innenhohlraum 2' des zentralen Implantatteils vorgelegte, verflüssigbare Material kann wie aussen am zentralen Implantatteil angeordentes, verflüssigbares Material ein thermoplastisches Material sein oder vorteilhafterweise ein hochviskoser polymerer oder hydraulischer Zement mit thixotropen Eigenschaften, der nach der Implantation beispielsweise durch ultraviolettes Licht, Wärme, mechanische Schwingungen oder einfach durch Zeit aushärtbar ist.

Bei Verwendung eines Thermoplasten als im Innenhohlraum 2' des zentralen Implantatteils 1 vorgelegtes, verflüssigbares Material sind an den Innenoberflächen des zentralen Implantatteils 1 oder an den Oberflächen des Thermoplasten gegebenenfalls Energierichtungsgeber anzuordnen.

Das verflüssigbaren Material kann auch für ein Implantat gemäss Figuren 11 und 12 herstellerseitig im zentralen Implantatteil 1 vorgelegt werden. Es kann aber auch vom Chirurgen in einer beliebigen Zahl von einzelnen Portionen vorgelegt werden oder es kann durch die Sonotrode hindurch im wesentlichen kontinuierlich in den zentralen Implantatteil 1 eingepresst werden.

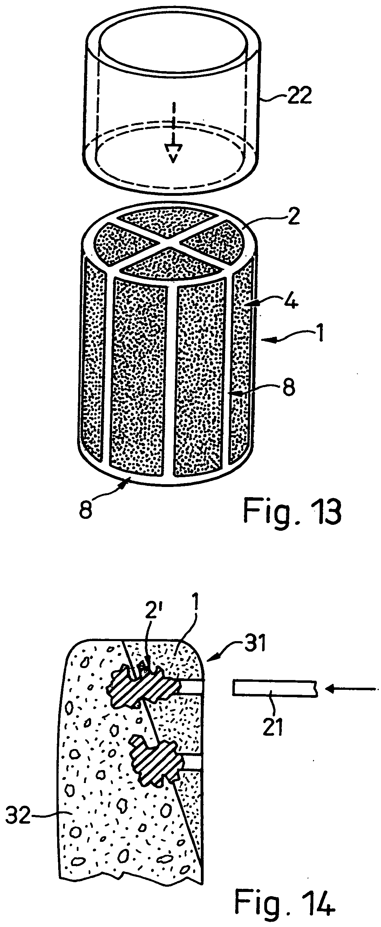

**Figur 13** zeigt eine weitere, beispielhafte Ausführungsform des erfindungsgemässen Implantates, das im Gegensatz zu den in den vorgehenden Figuren gezeigten Implantaten keine lasttragende Funktion übernimmt, dessen Funktion also beispielsweise die Freisetzung eines therapeutisch wirkenden Stoffes, eine Drainage oder eine elektrische oder chemische Stimulation von Gewebe oder Organen oder ähnliches ist.

Der periphere Implantatteil 2, der mindestens teilweise aus dem verflüssigbaren Material besteht (Oberflächenbereiche 8 der zweiten Art) ist käfigartig ausgebildet, wobei dieser Käfig eine für die Implantation genügende mechanische Stabilität aufzuweisen hat. Der zentrale Implantatteil 1, der keine lasttragende Funktion übernehmen muss, ist in diesem Käfig angeordnet. Das Implantat wird in einer Öffnung im Knochengewebe positioniert und dann mit einer an die proximale Stirnseite des Implantates angepassten Vorrichtung (Sonotrode eines Ultraschallgerätes) mit Schwingungsenergie beaufschlagt. Für das dargestellte Implantat ist die Sonotrode 22 beispielsweise hohlzylinderförmig.

Der zentrale Implantatteil 1 des Implantates gemäss Figur 14, der die Oberflächenbereiche 4 der ersten Art bildet, hat beispielsweise eine osseointegrative Funktion und besteht beispielsweise aus einem hochporösen Calziumphosphat, aus Knochenspan (patienteneigene Spongiosa) oder aus einem Gel. Es kann sich aber auch um eine Vorrichtung handeln, durch die Partikel oder Moleküle in die Umgebung abgegeben (delivery device) oder aus der Umgebung aufgenommen (Drainagevorrichtung) oder um einen Stimulator handeln, wobei diese Vorrichtung beispielsweise als entsprechend durchlässiger Behälter ausgebildet ist und die Behälterwände die Oberflächenbereiche 4 erster Art bilden.

Der Käfig gemäss Figur 13 kann herstellerseitig mit einem zentralen Implantatteil versehen werden oder im Operationssaal beispielsweise mit Knochenspan gefüllt werden. Es ist auch denkbar, den Käfig leer zu implantieren und in situ mit einem zentralen Implantatteil zu versehen, wobei ein den zentralen Implantatteil haltendes Deckelelement mittels Ultraschallverschweissung ebenfalls in situ angebracht werden kann.

**Figur 14** zeigt ein Augmentationselement 31, das zur Erzeugung von zusätzlich zum natürlichen Knochengewebe erwünschtem Knochengewebe, beispielsweise für die Verbreiterung eines Kieferkammes 32 verwendbar ist. Der Kieferkamm 32 und das Augmentationselement 31 sind im Schnitt nach der Implantation dargestellt. Das Augmentationselement 31 weist wiederum einen zentralen Implantatteil 1 auf, der aus einem das Knochenwachstum fördernden Material, beispielsweise aus einem hochporösen Calziumphosphat besteht. In beispielsweise durchgehenden Öffnungen (Innenhohlräume 2') des zentralen Implantatteils 1 sind Stifte des verflüssigbaren Materials angeordnet. Für die Implantation wird das Augmentationselement 31 am entsprechend präparierten Kieferkamm 32 positioniert, derart, dass die Stifte beispielsweise gegen den Kieferkamm 32 gerichtet sind. Dann wird mit einer auf den Stiftquerschnitt abgestimmten Sonotrode 21 Schwingungsenergie in die Stifte eingekoppelt und werden diese gegen den Kieferkamm 32 gepresst. Dadurch wird das verflüssigbare Material mindestens teilweise verflüssigt und in den Kieferkamm und den zentralen Implantatteil gepresst, wodurch das Augmentationselement 31 punktuell am Kieferkamm 32 befestigt ist und der zentrale Implantationsteil (Oberflächenbereiche der ersten Art) mit dem Knochengewebe des Kieferkamms in intensivem Kontakt steht, so dass schon unmittelbar nach der Implantation Zellen aus dem natürlichen Knochen in den zentralen Implantatteil einwandern und knochenbildend wirken können. Das verflüssigbare Material ist im vorliegenden Falle vorteilhafterweise resorbierbar.

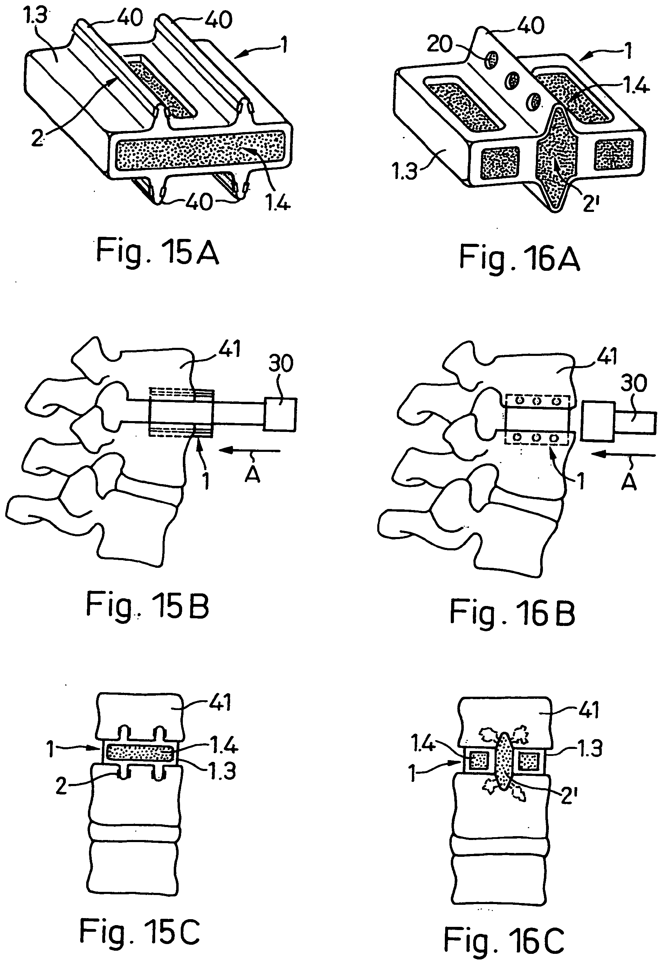

**Die** **Figuren 15** **A bis 15C und 16A bis 16C** zeigen zwei Implantate, die zur Verbindung von zwei Wirbelkörpern verwendbar sind. Die Implantate weisen wiederum einen zentralen Implantationsteil 1 auf, der hier ein lasttragendes Gerüst 1.3 und einen in diesem Gerüst angeordneten, für die Durchwachsung mit Knochengewebe ausgerüsteten Füllkörper 1.4, beispielsweise aus hochporösem Calziumphosphat, aus Knochenspan oder aus einem Gel aufweist. Der zentrale Implantatteil ist in seiner Form an eine natürliche Bandscheibe angepasst und weist unten und oben einen oder mehrere Kämme 40 auf, die in Implantationsrichtung A verlaufen und in entsprechende, in den Wirbelkörpern 41 zu erstellende Nuten passen.

Der periphere Implantatteil 2 ist in der Ausführungsform gemäss Figuren 16 auf den Kämmen 40 angeodnet und gemäss Figuren 16 als in Innenhohlräumen 2' des zentralen Implantatteils 1 vorgelegt, wobei im Bereiche der Kämme 40 Öffnungen 20 vorgesehen sind.

Das Implantat gemäss Figur 15A wird, wie in der Figur 15B dargestellt, mittels Sonotrode 30 zwischen zwei entsprechend präparierte Wirbelkörper 41 getrieben, wobei sich das verflüssigbare Material des peripheren Implantatteils 2 verflüssigt und in das Knochengewebe der Wirbelkörper gepressst und das Implantat dadurch in diesen verankert wird, wie dies in der Figur 15C dargestellt ist. Die zu verwendende Sonotrode 30 ist im wesentlichen auf die proximale Stirnseite des Implantates abgestimmt.

Das Implantat gemäss Figur 16A, welches nicht Teil der Erfindung ist, wird wie in der Figur 16B dargestellt zwischen den Wirbelkörpern 41 positioniert, beispielsweise mit einer Sonotrode 30, die im wesentlichen auf die proximale Stirnseite des lasttragenden Gerüstes 1.3 des zentralen Implantatteils 1 abgestimmt ist. Wenn das Implantat positioniert ist, wird mit einer Sonotrode, die auf die proximale Stirnseite des Innenhohlraumes 2' abgestimmt ist, Schwingungsenergie in dieses Material eingekoppelt, wodurch das Material durch die Öffnungen 20 und in das Knochengewebe der Wirbelkörper 41 gepresst wird und so das Implantat in den Wirbelkörpern verankert, wie dies in der Figur 16C dargestellt ist.

Die Implantate gemäss Figuren 15 und 16 sind unmittelbar nach der Implantation mit den Wirbelkörpern 41 fest verbunden (primäre Stabilität), so dass sich eine Fixierung der beteiligten Wirbel, wie sie gemäss dem Stande der Technik notwendig ist, erübrigt. Dadurch eignen sich die Implantate ausgezeichnet für minimal invasive Eingriffe.

## Patentansprüche

1. Implantat zur Implantation in menschlichem oder tierischem Knochengewebe oder durch Knochenersatzmaterial ergänztes Knochengewebe, wobei mindestens ein Teil der Implantatoberfläche mit dem Knochengewebe in Kontakt kommt,
- wobei das Implantat einen zentralen Implantatteil (1) sowie einen aussen am zentralen Implantatteil (1) angeordneten, peripheren Implantatteil (2) aufweist,
- wobei der genannte Teil der Implantatsoberfläche vom zentralen Implantatteil (1) gebildete Oberflächenbereiche (4) einer ersten Art und vom peripheren Implantatteil (2) gebildete, von den Oberflächenbereichen (4) der ersten Art verschiedene Oberflächenbereiche (8) einer zweiten Art aufweist,
- wobei die wobei die Oberflächenbereiche (4, 8) erster und zweiter Art sich im Wesentlichen parallel zu einer Implantationsrichtung (A) erstreckend nebeneinander angeordnet sind,
- wobei die Oberflächenbereiche (8) zweiter Art ein mittels mechanischer Schwingungen verflüssigbares Material aufweisen, mit dessen Hilfe das Implantat bei einer Implantation mittels mechanischer Schwingungen im Knochengewebe mindestens primär stabilisierbar ist,
- wobei die Oberflächenbereiche (4) erster Art für eine von einer primären Stabilisierung verschiedene, klinische Funktion ausgerüstet sind,
- wobei die Oberflächenbereiche (4,8) erster und zweiter Art derart dimensioniert und angeordnet sind, dass die Oberflächenbereiche (4) erster Art bei der Implantation mittels mechanischer Schwingungen mindestens teilweise von verflüssigtem Material frei bleiben,
- und wobei zwischen dem zentralen Implantatteil (1) und dem peripheren Implantatteil (2) eine formschlüssige Verbindung vorhanden ist.

2. Implantat nach Anspruch 1, **dadurch gekennzeichnet, dass** die von der primären Stabilisierung verschiedene, klinische Funktion der Oberflächenbereiche (4) der ersten Art eine Förderung der Osseointegration oder ein Durchlassen von Partikeln oder Molekülen aus dem Implantat in das Implantat umgebendes Knochengewebe oder aus das Implantat umgebendem Knochengewebe in das Implantat oder eine elektrische oder chemische Stimulation ist.

3. Implantat nach Anspruch 1 oder 2, **dadurch gekennzeichnet, dass** das mittels mechanischer Schwingungen verflüssigbare Material ein Material mit thermoplastischen Eigenschaften oder mit thixotropen Eigenschaften ist.

4. Implantat nach Anspruch 3, **dadurch gekennzeichnet, dass** das mittels mechanischer Schwingungen verflüssigbare Material ein Polymer auf Milch- und/oder Glykolsäurebasis, ein Polyhydroxyalkanoat, ein Polycaprolactone, ein Polysacharid, ein Polypeptid, ein Polydioxanon, ein Polyanhydrid, ein Polyolefin, ein Polyacrylat, ein Polymetacrylat, ein Polycarbonat, ein Polyamid, ein Polyester, ein Polyurethan, ein Polysulfon, ein Polyarylketon, ein Polyimid, ein Polyphenylsulfid, ein Flüssig-Kristall-Polymer, ein Polyacetal, ein halogeniertes Polymer, insbesondere ein halogeniertes Polyolefin, ein Polyphenylensulfid, ein Polysulfon oder ein Polyether ist oder ein Copolymer oder Mischpolymer der genannten Polymere oder ein Verbundwerkstoff, der eines der genannten Polymere enthält, oder ein polymerer, keramischer oder hydraulischer Zement.

5. Implantat nach Anspruch einem der Ansprüche 1 bis 4, **dadurch gekennzeichnet, dass** die Oberflächenbereiche (4) erster Art eine Osteointegrations-fördernde Funktion haben und Oberflächenstrukturen aufweisen, die sich für eine Verwachsung oder Durchwachsung mit vitalem Knochengewebe eignen, und dass die Oberflächenbereiche (4) erster Art beispielsweise zusätzlich entzündungshemmende, infektionsbekämpfende und/oder wachstumsfördernde Eigenschaften haben.

6. Implantat nach einem der vorangehenden Ansprüche, **dadurch gekennzeichnet, dass** die Oberflächenbereiche (8) der zweiten Art mindestens örtlich über die Oberflächenbereiche (4) der ersten Art vorstehen.

7. Implantat nach einem der vorangehenden Ansprüche, **dadurch gekennzeichnet, dass** das Implantat eine lasttragende Funktion hat und dass der zentrale Implantatteil (1) das lasttragende Element darstellt.

8. Implantat nach Anspruch 7, **dadurch gekennzeichnet, dass** der zentrale Implantatteil (1) mindestens teilweise aus einem Metall, einer Metalllegierung, aus einem keramischen oder glasartigen Material, aus einem Kunststoff oder aus einem Verbundwerkstoff besteht und beispielsweise selbstschneidende oder furchende Elemente aufweist.

9. Implantat nach Anspruch 7 oder 8, **dadurch gekennzeichnet, dass** der zentrale Implantatteil (1) einen lasttragenden Teil (1.1) und einen formvariablen Teil (1.2) aufweist.

10. Implantat nach Anspruch 1, **dadurch gekennzeichnet, dass** es als Dentalimplantat mit mindestens einer Fixierstelle (3) oder mit mindestens einem Kronenteil ausgebildet ist, oder dass es für eine orthopädische Anwendung ausgebildet ist.

11. Implantat nach Anspruch 10, **dadurch gekennzeichnet, dass** es stift-, platten- oder scheibenförmig ist oder eine an eine vorgegebenen Kavität in einem Knochen angepasste oder anpassbare Form hat.

12. Implantat nach Anspruch 10, **dadurch gekennzeichnet, dass** es ein Implantat zur Verbindung von zwei Knochenteilen oder zur Fixierung einer Stützplatte ist oder der Schaft einer Prothese für ein Hüftgelenk, ein Fingergelenk, ein Kniegelenk oder ein Schultergelenk.

13. Implantat nach einem der Ansprüche 1 bis 8, **dadurch gekennzeichnet, dass** es ankerförmig ausgebildet ist und eine als Öse ausgebildete Fixierstelle (3) aufweist.

## Claims

1. An implant for implantation in human or animal bone tissue or bone tissue supplemented by bone replacement material, wherein at least a part of the implant surface comes into contact with the bone tissue,
- wherein the implant comprises a central implant part (1) as well as a peripheral implant part (2) which is arranged at the outside on the central implant part (1),
- wherein the mentioned part of the implant surface comprises surface regions (4) of a first type which are formed by the central implant part (1), and surface regions (8) of a second type which are formed by the peripheral implant part (2) and which are different from the surface regions (4) of the first type,
- wherein the surface regions (4, 8) of the first and second type are arranged next to one another extending essentially parallel to an implantation direction (A),
- wherein the surface regions (8) of the second type comprise a material which is liquefiable by way of mechanical oscillations and with the help of which the implant can be stabilised in the bone tissue at least primarily by way mechanical oscillations on implantation,
- wherein the surface regions (4) of the first type are designed for a clinical function which is different to a primary stabilisation,
- wherein the surface regions (4, 8) of the first and second type are dimensioned and arranged such that the surface regions (4) of the first type at least partly remain free of liquefied material, on implantation by way of mechanical oscillations,
- and wherein a positive connection is present between the central implant part (1) and the peripheral implant part (2).

2. An implant according to claim 1, **characterised in that** the clinical function of the surface regions (4) of the first type and which is different from the primary stabilisation is a promotion of osseointegration or a passage of particles or molecules out of the implant into bone tissue surrounding the implant or out of bone tissue surrounding the implant into the implant, or an electrical or chemical stimulation.

3. An implant according to claim 1 or 2, **characterised in that** the material which is liquifiable by way of mechanical oscillations is a material with thermoplastic characteristics or with thixotropic characteristics.

4. An implant according to claim 3, **characterised in that** the material which can be liquefied by way of mechanical oscillations is a polymer based on lactic acid and/or glycolic acid, a polyhydroxyalkanoate, a polycaprolactone, a polysaccharide, a polypeptide, a polydioxanone, a polyanhydride, a polyolefin, a polyacrylate, a polymetacrylate, a polycarbonate, a polyamide, a polyester, a polyurethane, a polysulphone, a polyarylketone, a polyimide, a polyphenyl sulphide, a liquid crystal polymer, a polyacetal, a halogenated polymer, in particular a halogenated polyolefin, a polyphenyl sulphide, a polysulphone or a polyether, or a copolymer or a mixed polymer of the mentioned polymers or a composite material which comprises one of the mentioned polymers, or a polymeric, ceramic or hydraulic cement.

5. An implant according to one of the claims 1 to 4, **characterised in that** the surface regions of the first type have a function which promotes osteointegration and comprise surface structures which are suitable for an intergrowth or through-growth with vital bone tissue, and that the surface regions (4) of the first type for example additionally have antiinflammatory, infection-combatting and/or growth-promoting characteristics.

6. An implant according to one of the preceding claims, **characterised in that** the surface regions (8) of the second type at least locally project beyond the surface regions (4) of the first type.

7. An implant according to one of the preceding claims, **characterised in that** the implant has a load-bearing function and **in that** the central implant part (1) represents the load-supporting element.

8. An implant according to claim 7, **characterised in that** the central implant part (1) at least partly consists of metal, of a metal alloy, of a ceramic or glass-like material, of a plastic or of a composite material and for example comprises self-cutting or furrowing elements.

9. An implant according to claim 7 or 8, **characterised in that** the central implant part (1) comprises a load-bearing part (1.1) and a shape-variable part (1.2).

10. An implant according to claim 1, **characterised in that** it is designed as a dental implant with at least one fixation location (3) or with at least one crown part, or that it is designed for an orthopaedic application.

11. An implant according to claim 10, **characterised in that** it is pin-like, plate-like or disc-like or has a shape which is adapted or adaptable to a defined cavity in a bone.

12. An implant according to claim 10, **characterised in that** it is an implant for the connection of two bone parts or for fixing a support plate, or the shank of a prosthesis for a hip joint, a finger joint, a knee joint or a shoulder joint.

13. An implant according to one of the claims 1 to 8, **characterised in that** it is designed in the manner of an anchor and comprises a fixation location (3) formed as an eyelet.

## Revendications

1. Implant destiné à être implanté dans un tissu osseux humain ou animal ou dans un tissu osseux complété par un matériau de remplacement osseux,
au moins une partie de la surface de l'implant entrant en contact avec le tissu osseux,
l'implant présentant une partie centrale (1) d'implant et une partie périphérique (2) d'implant disposée par l'extérieur sur la partie centrale (1) de l'implant,
ladite partie de la surface de l'implant présentant des parties de surface (4) d'un premier type formées par la partie centrale (1) de l'implant et des parties de surface (8) d'un deuxième type, différentes des parties de surface (4) du premier type, formées par la partie périphérique (2) de l'implant,
les parties de surface (4, 8) du premier et du deuxième type étant disposées de manière à s'étendre les unes à côté des autres essentiellement en parallèle à la direction d'implantation (A),
les parties de surface (8) du deuxième type présentant un matériau apte à être liquéfié sous l'action de vibrations mécaniques et à l'aide duquel l'implant peut être stabilisé de manière au moins primaire dans le tissu osseux au moyen de vibrations mécaniques appliquées lors de l'implantation,
les parties de surface (4) du premier type étant prévues pour assurer une fonction clinique différente de la stabilisation primaire,
les parties de surface (4, 8) du premier et du deuxième type étant dimensionnées et disposées de telle sorte que les parties de surface (4) du premier type restent au moins en partie exemptes de matériau liquéfié au moyen de vibrations mécaniques lors de l'implantation et
une liaison à engagement positif étant prévue entre la partie centrale (1) de l'implant et la partie périphérique (2) de l'implant.

2. Implant selon la revendication 1, **caractérisé en ce que** la fonction clinique des parties de surface (4) du premier type, différente de la stabilisation primaire, est une promotion de l'intégration osseuse, le passage de particules ou de molécules de l'implant jusque dans le tissu osseux qui entoure l'implant ou de tissu osseux entourant l'implant jusque dans l'implant, ou une stimulation électrique ou chimique.

3. Implant selon les revendications 1 ou 2, **caractérisé en ce que** le matériau liquéfiable sous l'action de vibrations mécaniques est un matériau qui présente des propriétés thermoplastiques ou des propriétés thixotropiques.

4. Implant selon la revendication 3, **caractérisé en ce que** le matériau liquéfiable sous l'action de vibrations mécaniques est un polymère à base d'acide lactique et/ou d'acide glycolique, un polyhydroxyalcanoate, un polycaprolactone, un polysaccharide, un polypeptide, un polydioxanone, un polyanhydride, une polyoléfine, un polyacrylate, un polyméthacrylate, un polycarbonate, un polyamide, un polyester, un polyuréthane, un polysulfone,
une polyarylcétone, un polyimide, un poly(sulfure de phényle), un polymère à cristaux liquides, un polyacétal, un polymère halogéné et en particulier une polyoléfine halogénée, un poly(sulfure de phénylène), un polysulfone, un polyéther, ou un copolymère ou polymère mixte desdits polymères, un matériau composite qui contient l'un desdits polymères, ou un ciment polymère, céramique ou hydraulique.

5. Implant selon l'une des revendications 1 à 4, **caractérisé en ce que** les parties de surface (4) du premier type ont une fonction prolongeant l'intégration osseuse et présentent des structures de surface qui conviennent pour la croissance ou l'imprégnation par du tissu osseux vital et **en ce que** les parties de surface (4) du premier type ont par exemple des propriétés supplémentaires anti-inflammatoires, de lutte contre les infections et/ou de promotion de la croissance.

6. Implant selon l'une des revendications précédentes, **caractérisé en ce que** les parties de surface (8) du deuxième type débordent au moins localement au-delà des parties de surface (4) du premier type.

7. Implant selon l'une des revendications précédentes, **caractérisé en ce que** l'implant a une fonction de reprise des charges et **en ce que** la partie centrale (1) de l'implant constitue un élément de reprise de charge.

8. Implant selon la revendication 7, **caractérisé en ce que** la partie centrale (1) de l'implant est constituée au moins en partie d'un métal, d'un alliage métallique, d'un matériau céramique ou vitreux, d'une matière synthétique ou d'un matériau composite et présente par exemple des éléments auto-coupants ou taraudeurs.

9. Implant selon les revendications 7 ou 8, **caractérisé en ce que** la partie centrale (1) de l'implant présente une partie (1.1) de reprise de charge et une partie (1.2) de forme variable.

10. Implant selon la revendication 1, **caractérisé en ce qu'**il est configuré comme implant dentaire présentant au moins un emplacement de fixation (3) ou au moins une partie de couronne, ou **en ce qu'**il est configuré pour une application orthopédique.

11. Implant selon la revendication 10, **caractérisé en ce qu'**il est en forme de tige, de plaque ou de disque ou a une forme adaptée ou adaptable à une cavité prédéterminée d'un os.

12. Implant selon la revendication 10, **caractérisé en ce qu'**il est un implant destiné à relier deux parties d'os ou à fixer une plaque de soutien, ou la tige d'une prothèse d'articulation de la hanche, d'articulation du doigt, d'articulation du genou ou d'articulation de l'épaule.

13. Implant selon l'une des revendications 1 à 8, **caractérisé en ce qu'**il est configuré en forme d'ancre et présente un emplacement de fixation (3) configuré en oeillet.
